# EUROPÄISCHE PATENTSCHRIFT

(11) **EP 0 599 064 B1**
(45) Veröffentlichungstag und Bekanntmachung des Hinweises auf die Patenterteilung: **06.05.1998**
(21) Anmeldenummer: 93117141.7
(22) Anmeldetag: 22.10.1993
(51) Int. Cl.: A61K 9/50, A61K 9/28

(54) **Retard-Form für pharmazeutische Wirkstoffe**
Retard form for pharmaceutically active agents
Forme retard pour principes actifs pharmaceutiques

(30) Priorität: 21.11.1992 DE 4239244
(43) Veröffentlichungstag der Anmeldung: 01.06.1994
(73) Patentinhaber: BASF Aktiengesellschaft, 67063 Ludwigshafen (DE)
(72) Erfinder: Harth, Klaus, Dr., D-67317 Altleiningen (DE); Hibst, Hartmut, Dr., D-69198 Schriesheim (DE); Dembowski, Juergen, Dr., D-67307 Goellheim (DE); Spengler, Reinhard, Dr., D-67071 Ludwigshafen (DE); Flaig, Ernst, Dr., D-69121 Heidelberg (DE)

(56) Entgegenhaltungen:
- WO-A-90/02546
- ADV. LOW-TEMP. PLASMA CHEM. TECHNOL. APPL. Bd. 1 , 1984 Seiten 339 - 342 N. HASIRCI ET AL. 'some studies on coating of activated charcoal with plasma polymer hexamethyldisiloxane'

## Beschreibung

Die Erfindung betrifft eine neuartige, extrem dünne Retardbeschichtung für feste pharmazeutische Formen, die eine bisher nicht erreichte Genauigkeit bei der Einstellung der Wirkstofffreigabegeschwindigkeit zuläßt.

Bei der Behandlung von Krankheiten über einen längeren Zeitraum ist es wünschenswert, die Häufigkeit der Einnahme der Medikamente möglichst gering zu halten. Dies verbessert die Behandlungssicherheit durch die Vermeidung unregelmäßiger Einnahmen, indem es die vorhandene Wirkstoffkonzentration im Körper stabilisiert und so das Risiko unerwünschter Über- bzw. Unterdosierungen reduziert. Darüber hinaus erleichtert es die Behandlung für den Patient.

Es werden daher in vielen Fällen Zubereitungsformen für die Medikamente gesucht, die eine kontrollierte Freigabe des pharmazeutischen Wirkstoffes über einen längeren Zeitraum ermöglichen. Im idealen Falle sollte eine solche Retardzubereitung eine in weiten Grenzen genau einstellbare Freigabe-Verzögerung sowie einen hohen Wirkstoffgehalt erlauben.

Es sind verschiedene Verfahren zur Herstellung von Retard-Zubereitungen für die orale Darreichung von Medikamenten bekannt.

Bei Gerüsttabletten wird der Wirkstoff in eine Matrix aus geeigneten Hilfsstoffen eingebettet (vgl. Ullmanns Encyklopädie der technischen Chemie, Band 18, Pharmazeutische Technologien). Aus derartigen Gerüsttabletten wird der Wirkstoff durch Diffusion an die Oberfläche des gequollenen und/oder porösen Tablettenkörpers langsam freigesetzt.

Die durch Einbettung eines Wirkstoffes in eine Matrix erzielbare Retardwirkung ist nur bedingt steuerbar. Der mit zunehmender Zeit ansteigende Diffusionsweg bedingt dabei eine in der Regel entsprechend einer Exponentialfunktion abfallende Freisetzungsgeschwindigkeit des Wirkstoffes. Darüber hinaus führt der hohe Anteil der Hilfsstoffe an der Gesamtmenge der Gerüsttablette zu einer Beschränkung der verabreichbaren Wirkstoffmenge.

Eine Retardierung der Wirkstofffreigabe kann auch durch eine physikalisch chemische Bindung des Wirkstoffes an eine geeignete Trägersubstanz, wie biologisch abbaubare Polymere oder Ionenaustauscher, erreicht werden, wobei die Freisetzungsrate durch den Abbau der Trägersubstanz oder den Ioneneinfluß bestimmt wird. Diese Systeme sind in der Regel jedoch nur für relativ niedrig dosierte Wirkstoffe geeignet.

Ein weit verbreitetes Verfahren zur Herstellung von Retardformen ist die Lackierung wirkstoffhaltiger Kerne mit geeigneten Lacken aus z.B. den Estern von Acrylaten und Methacrylaten oder Cellulose-Derivaten wie Ethylcellulose. Die Wirkstofffreigabe wird dabei durch die Diffusion des Arzneistoffes durch den als Membran wirkenden Überzug gesteuert.

Die Herstellung der genannten Lacküberzüge erfolgt üblicherweise (vgl. Bauer-Lehmann-Osterwald-Rothgang, Überzogene Arzneiformen, WVG, Stuttgart, 1988) durch Aufsprühen der Lackschicht in einem rotierenden Kessel oder einem Wirbelschichtreaktor und anschließende Trocknung. Zum Aufbringen der Lackschicht werden sowohl organische als auch wäßrige Lösungsmittel verwendet, die beide spezifische Nachteile besitzen: Organische Lösungsmittel verlangen aufwendige technische Maßnahmen zur Vermeidung von umweltbelastenden Emissionen und zur Gewährleistung der Arbeitssicherheit. Darüber hinaus besteht bei organischen Lösungsmitteln die Gefahr von Rückständen im Medikament, die toxische Nebenwirkungen zur Folge haben können. Wäßrige Lösungsmittel sind zwar unbedenklich für die Umwelt und hinsichtlich toxischer Rückstände, ihre Verwendung führt jedoch in der Regel zu einer schlechteren Homogenität des Lacküberzugs. Um einer hierdurch bedingten Beeinträchtigung der Retardwirkung entgegenzuwirken, muß daher eine Erhöhung der Lackschichtdicke in Kauf genommen werden.

US 3 916 899 beschreibt eine Retardierungsform für Wirkstoffe, bei welcher das Wirkstoffreservoir (z.B. in Form einer Kapsel) von einer semipermeablen Lackschicht umgeben wird, welche für den Wirkstoff undurchlässig, für die jeweilige Flüssigkeit des Umgebungsmediums jedoch permeabel ist. Die umgebende Lackschicht enthält zusätzlich eine geometrisch wohldefinierte Öffnung, durch welche der in der Umgebungsflüssigkeit gelöste Wirkstoff nach außen gelangen kann. Nach Einnahme der Retardform diffundieren die Verdauungssäfte durch die semipermeable Lackschicht hindurch ins Innere der Kapsel, wobei die Durchflußmenge durch die über die Lackschicht abfallende osmotische Druckdifferenz sowie durch die Permeabilität der Lackschicht geregelt wird. Der Wirkstoff im Innern der Kapsel wird in der einströmenden Flüssigkeit kontinuierlich gelöst und gelangt über die geometrische Öffnung in der Lackschicht mit definierter Rate nach außen. Bei dem erwähnten osmotischen System kann es allerdings im Magen- oder Darmtrakt je nach eingesetzter Kapselfüllung zu lokalen Irritationen des Gewebes durch eine erhöhte Konzentration kommen. Ein Teil des Wirkstoffes verbleibt außerdem in der Regel in der Arzneiform und steht somit nicht für die gewünschte Resorption zur Verfügung. Die Herstellung der genannten osmotischen Retardierungsformen ist sehr aufwendig, da die Lackschicht jeder einzelnen Kapsel mit geeigneten Methoden, beispielsweise mit Hilfe eines Laserstrahls, durchbohrt werden muß.

Es bestand daher die Aufgabe, eine neue, hochpräzise Retardform für pharmazeutische Wirkstoffe auf der Basis eines diffusionshemmenden Überzugs mit folgenden vorteilhaften Kennzeichen zu finden:
- gute Retardwirkung auch bei kleinen Schichtdicken (< 1 µm)
- hochpräzises, über die Schichtdicke steuerbares Freisetzungsverhalten
- reproduzierbares, homogenes Freisetzungsverhalten von Pellet zu Pellet
- weitgehender Verzicht auf sonstige galenische Hilfsmittel, so daß sehr hohe Wirkstoffgehalte ermöglicht werden
- lösungsmittelfreies, toxikologisch unbedenkliches, emissionsfreies Herstellungsverfahren für die Retardschicht.

Die Lösung der Aufgabe besteht in einer Retardform und dem Verfahren zu deren Herstellung gemäß den Ansprüchen.

Wesentliches Kennzeichen der neuen Retardform ist die Verwendung einer dünnen, die Diffusion hemmenden Deckschicht, die sich in Aufbau, chemischer Zusammensetzung, Herstellung und Exaktheit der Einstellbarkeit der Wirkung sehr stark von den bisher bekannten, die Diffusion bzw. Auflösung hemmenden Überzügen unterscheidet.

Die erfindungsgemäßen Schichten besitzen eine sehr gute diffusionshemmende Wirkung bereits bei einer Schichtdicke deutlich unterhalb von 1 µm. Damit wird eine erhebliche Reduktion der Überzugsdicke gegenüber dem Stand der Technik erreicht. Die erfindungsgemäßen Retardschichten gewährleisten eine sehr homogene, zeitlich lineare Freisetzung des Wirkstoffes. Beliebige Retardwirkungen können somit präzise in einfacher Weise durch die Wahl einer geeigneten Schichtdicke eingestellt werden. Dabei besteht eine große Freiheit hinsichtlich der Art und der Form der wirkstoffhaltigen Partikel. Insbesondere ermöglicht die erfindungsgemäße Retardform die Verwendung hochkonzentrierter Wirkstoffträger, die eine besonders lang anhaltende Wirkstofffreigabe gewährleisten.

Wichtige Kennzeichen der erfindungsgemäßen Retardschichten sind zum einen die spezielle chemische Zusammensetzung der Schicht, die durch einen hohen Vernetzungsgrad geprägt ist, und zum anderen die neuartige Herstellung der Schicht in einem plasmaunterstützten chemischen Abscheideverfahren.

Hinsichtlich der chemischen Zusammensetzung wurde gefunden, daß eine besonders gute Retardwirkung erhalten wird, wenn die Retardschicht mindestens die Elemente Si und C enthält, wobei der Gehalt (bezogen auf die Gesamtmenge aller Elemente ohne H) von Silizium bevorzugt im Bereich von 1 bis 40 Atom-% und von Kohlenstoff im Bereich von 60 bis 99 Atom-% liegt. Die Schicht kann auch Sauerstoff, Stickstoff und vor allem Wasserstoff enthalten. Geringe Mengen weiterer Elemente stören im allgemeinen nicht. Unabdingbar zur Erzielung der gewünschten Hemmwirkung ist eine starke chemische Vernetzung der C-Atome, vorzugsweise der Si- und C-Atome der Schicht. Diese Vernetzung führt dazu, daß die Si-Atome auch bei relativ hohen O-Gehalten der Schicht durch einen "metallischen" Bindungstyp und die C-Atome gleichzeitig durch einen "carbidischen" Bindungstyp gekennzeichnet sind. Die erfindungsgemäßen Schichten unterscheiden sich damit wesentlich von anderen bekannten Diffusionshemmschichten, wie beispielsweise SiO₂-Schichten, bei welchen die Si-Atome einen "oxidischen" Bindungstyp aufweisen. Der genannte Charakter des Bindungstyps kann durch eine ESCA (electron spectroscopy for chemical analysis)-Untersuchung der Schichten experimentell bestimmt werden.

Als vorteilhaftes Verfahren für die Herstellung der erfindungsgemäßen Retardformen wurde eine spezielle plasmaunterstützte chemische Abscheidung eines Gemisches, welches Si-organische Monomere enthält, gefunden.

Plasmaunterstützte Vakuumprozesse zur Erzeugung dünner Schichten sind seit längerem bekannt (vgl. R.F. Bhunshah et al, "Deposition Technologies for Films and Coatings", Noyes Publications, 1982; H. Yasuda, "Plasma Polymerization", Academic Press, Orlando, 1985) und finden bereits in vielen Bereichen industrielle Anwendung, beispielsweise bei der Herstellung von Halbleiterbauelementen, von magnetischen oder optischen Datenträgern oder von Verschleißschutzschichten für metallische Werkzeuge. Dabei unterscheidet man in der Regel physikalische Depositionsverfahren (sog. PVD-Verfahren, wie z.B. Aufdampfen, Kathodenzerstäubung (Sputtern) oder Ionenplattieren), bei welchen das Ausgangsmaterial in kondensierter Form vorliegt, von chemischen Depositionsverfahren (sog. CVD-Verfahren), bei welchen eine geeignete gasförmige Ausgangsverbindung in den Beschichtungsraum eingeführt, zersetzt und dabei in Form einer dünnen Schicht abgeschieden wird. Die Zersetzung der gasförmigen Ausgangsverbindung kann dabei entweder durch thermische Energiezufuhr (thermisch CVD) oder unter Einwirkung eines Plasmas (plasmaunterstützte CVD) erfolgen. Die thermische CVD gasförmige Monomere verlangt in der Regel Zersetzungstemperaturen von mehreren hundert Grad Celsius und ist daher für die Beschichtung temperaturempfindlicher Materialien, wie pharmazeutischer Wirkstoffe, nicht geeignet.

Es sind bisher mehrere kommerzielle Anwendungen von plasmaunterstützten PVD- und CVD-Verfahren zur Herstellung von Diffusionssperrschichten bekannt, wobei jedoch ausnahmslos großflächige, in der Regel flache Träger mit möglichst glatten Oberflächen beschichtet werden. Die bekannten Verfahren zur Diffusionssperrbeschichtung versagten jedoch bisher bei der Beschichtung kleiner dreidimensionaler Träger mit mikroskopisch rauhen Oberflächen. Besondere Probleme bereiteten dabei die ausreichende Haftung der Schicht auf dem Trägermaterial (insbesondere bei hohen Schichtdicken), die Homogenität der Beschichtung sowie die vollständige Fehlstellenfreiheit der Schicht. Versuche zur Diffusionssperrbeschichtung von Wirkstoff-Pellets mit C-freien (Si-O bzw. Si-N)-Schichten durch reaktives Sputtern erbrachten daher nicht den gewünschten Erfolg: Wärend bei Schichtdicken unterhalb von 250 nm keine meßbare Retardwirkung erzielt wurde, führten größere Schichtdicken zu einer Versprödung der Deckschicht mit anschließendem mechanischen Zerfall der Deckschicht im Auflösungsversuch. Es war daher umso überraschender, daß mit dem erfindungsgemäßen Verfahren gute Retardschichten auch auf mikroskopisch rauhen Oberflächen von Wirkstoff-Pellets hergestellt werden konnten.

Das erfindungsgemäße Verfahren zeichnet sich dadurch aus, daß die zu beschichtenden Wirkstoffträger in einer Vakuumanlage, welche eine Plasmavorrichtung enthält, mit mechanischen oder strömungsmechanischen Hilfsmitteln bewegt werden, wobei gleichzeitig ein Gasgemisch, welches mindestens eine organische Verbindung enthält, mit vorgegebenen Fluß- und Druckverhältnissen in den Plasmabereich eingelassen wird. Die organische Verbindung kann eine Si-organische Verbindung oder eine Kohlenwasserstoffverbindung sein. Die Plasmabedingungen (Fluß und Druck) werden so eingestellt, daß im Plasmabereich eine Zersetzung der organischen Verbindung unter gleichzeitiger Bildung eines Netzwerkes auf den Wirkstoffträgern stattfindet. Geeignete Si-organische Verbindungen sind beispielsweise Hexamethyldisiloxan, Hexamethyldisilazan, Tetraethylorthosilikat, Vinyltrimethylsilan sowie andere methyl-, vinyl-, phenyl- oder alkoxygruppenhaltige Siloxane, Silazane oder Silane. Besonders bevorzugt als Si-organische Verbindung wird Hexamethyldisiloxan. Das eingesetzte Gasgemisch kann darüber hinaus Edelgase wie He, Ne, Ar, Kr oder Xe, bevorzugt Ar, sowie O₂ oder N₂ enthalten. Ein bevorzugter Fluß des Gemisches liegt dabei im Bereich von 1 bis 10 000 sccm bei einem Druck im Bereich von 1 x 10⁻³ bis 100 mbar.

Zur elektrischen Versorgung des Plasmas können entweder Gleichstrom- (DC) oder Wechselstromgeneratoren eingesetzt werden, wobei die Wechselstromgeneratoren üblicherweise bei 13,56 MHz (RF) oder 2,45 GHz (Mikrowelle) arbeiten. Die Einkopplung der DC-Spannung in die Vakuumanlage erfolgt dabei in bekannter Weise (vgl. z.B. R.F. Bhunshah et al, "Deposition Technologies for Films and Coatings, Noyes Publications, 1982) über eine runde, stabförmige oder mit einer sonstigen geeigneten Geometrie versehene, isolierte Elektrode, wobei der restliche Teil der Vakuumanlage auf Erdpotential gehalten wird. Die Einkopplung der RF-Spannung erfolgt in vergleichbare Weise, wobei jedoch zur Maximierung der eingekoppelten und zur Minimierung der reflektierten elektrischen Leistung eine elektrische Abstimmeinheit (elektrisches Netzwerk aus Spulen und Kondensatoren) zwischen Generator und Elektrode verwendet wird. Die alternativ verwendbare Mikrowellenanregung erfolgt in bekannter Weise elektrodenlos, wobei außerhalb des Vakuums Hohl- oder Koaxialleiter zur Zuführung der elektrischen Leistung benutzt werden. Zur Einkopplung der Mikrowelle in den Plasmabereich sind verschiedene Anordnungen bekannt, beispielsweise eine Hornantenne, welche im Innern eine Quarzplatte als Vakuumabdichtung enthält. Alternativ kann die Mikrowelle auch mit Hilfe von Stabantennen, die isoliert und vakuumdicht in einer Grundplatte befestigt sind, aus einem externen Hohlleiter in das Innere der Vakuumanlage geführt werden. Die Plasmadichte im Vakuumbereich kann dabei zusätzlich in bekannter Weise durch Magnetfelder intensiviert werden, was in der Regel zu einer Erhöhung der Ladungsträgerdichte und einer Erhöhung der Beschichtungsrate führt.

Um eine gleichmäßige Beschichtung und damit eine einheitliche Retardwirkung zu erzielen, ist es notwendig die zu beschichtenden, wirkstoffhaltigen oder aus dem Wirkstoff bestehenden Trägermaterialien während der Beschichtung mit geeigneten mechanischen oder strömungsmechanischen Vorrichtungen in Bewegung zu halten. Geeignete mechanische Vorrichtungen hierfür sind beispielsweise periodisch bewegte Käfige, Trommeln, Schalen oder Rinnen, in welchen die zu beschichtenden Träger zu statistischen Bewegungen angeregt werden. Die mechanische Vorrichtung muß dabei geeignete Öffnungen zur Durchströmung der gasförmigen Monomere und zum Durchgriff des Plasmas besitzen. Alternativ ist es möglich, daß die zu beschichtenden Träger durch ein Wirbelbettverfahren in statistischer Bewegung gehalten werden.

Wichtig für die Erzielung einer möglichst hohen Beschichtungsrate sowie einer guten Homogenität der Beschichtung ist eine optimierte Gesamtanordnung des Gaseinlaßsystems, des Plasmabereiches und der Bewegungsvorrichtung für die Wirkstoffträger. Bei der Verwendung von flachen Elektroden mit runder oder rechteckiger Geometrie ist es dabei günstig, daß die Grundfläche der Bewegungsvorrichtung, auf welcher die Wirkstoffträger mechanisch angeregt werden, eine der Elektrode ähnliche Geometrie besitzt, so daß der durch die Form der Elektrode vorgegebene Plasmabereich möglichst effektiv ausgenutzt wird. Gleichzeitig sollte das Gaseinlaßsystem eine gleichmäßige Durchströmung des Plasmabereiches gewährleisten. Bei der Einkopplung des Plasmas über eine runde Elektrode erweist sich beispielsweise die Verwendung einer oder mehrerer übereinanderliegender Bewegungsvorrichtungen mit gemeinsamer zentrischer Achse als günstig. Die Gaszuführung erfolgt dabei in vorteilhafter Weise auf der Höhe einer jeden Bewegungsvorrichtung durch eine ringförmige Gasdusche, deren Innendurchmesser etwas größer als der Außendurchmesser der Bewegungsvorrichtung gewählt wird. Die Ansaugverhältnisse des Pumpsystems sollten dabei in bevorzugter Weise eine radiale Strömung des Gases vom Außenrand zum Zentrum der Bewegungsvorrichtung gewährleisten.

Die erfindungsgemäßen Retardschichten können auf Wirkstoffträgern unterschiedlichster Art und Form eingesetzt werden. Geeignete Wirkstoffträger sind Granulate, Einkristalle, auch relativ kompakte Kristall- oder Pulverteilchen-Agglomerate, vor allem Pellets und insbesondere Tabletten. Sie bestehen aus dem pharmazeutischen Wirkstoff, wozu auch Vitamine gehören, oder enthalten ihn neben einem Träger und ggf. anderen Hilfsstoffen. Auch Folien können (zwecks transdermaler Applikation) erfindungsgemäß beschichtet werden.

Geeignete pharmazeutische Wirkstoffe sind alle, die in den Verdauungssäften ausreichend löslich sind, um durch die Deckschicht zu diffundieren und im Körper ihre heilsame oder vorbeugende Wirkung zu entfalten.

Pharmazeutische Hilfsstoffe, die im Kern neben dem Wirkstoff enthalten sein können, sind beispielsweise Binde-, Gleit-, Formtrenn-, Fließregulier- und Konservierungsmittel sowie Füllstoffe, Weichmacher, Antioxidantien.

### Beispiel 1

Aus Pellets des Wirkstoffes Theophyllin wurde durch Siebung eine Fraktion mit einem Pelletdurchmesser zwischen 1,0 und 1,4 mm ausgewählt. Die Pellets wurden auf eine Bewegungsvorrichtung aufgelegt, die aus einem Stahl-Netz mit einer Maschenweite von 0,4 mm bestand, welches über eine elektromagnetisch angetriebene Rüttelvorrichtung zu Vibrationen angeregt wurde.

Die gesamte Anordnung wurde in eine zylinderförmige Vakuumanlage eingebracht, welche mit einem zweistufigen Pumpsystem (Turbomolekularpumpe und Drehschiebervorpumpe) auf einen Druck von 10⁻⁶ mbar evakuiert wurde. Danach wurde über eine ringförmige Gasdusche mit einem Innendurchmesser von 150 mm ein Gasgemisch aus Hexamethyldisiloxan (Hersteller: Fa. Merck, Reinheit >99 %) mit einem Partialdruck von 2 x 10⁻² mbar und Argon mit einem Partialdruck von 1 x 10⁻² mbar eingelassen. Bei konstanter Saugleistung des Pumpsystems betrug dabei der Fluß von Hexamethyldisiloxan 35,8 sccm (standard cubic centimeters per minute).

Eine horizontal unter dem Stahlnetz in einem Abstand von 56 mm angebrachte runde Stahlelektrode (Durchmesser 150 mm) wurde mit Hilfe eines RF-Generators mit einer Wechselspannung der Frequenz 13,56 MHz beaufschlagt. Die Leistungsabgabe des RF-Generators wurde auf 100 W geregelt, wobei sich auf der Stahlelektrode ein Potential von -570 V gegenüber einem geerdeten Bezugspunkt einstellte. Nach einer Beschichtungszeit von 14 min wurde der RF-Generator abgeschaltet und damit die Beschichtung beendet.

Die beschichteten Pellets wurden in einer Apparatur Paddel Model USP XXI mit Patchholder bei einer Umdrehungszahl von 50 UpM und einer Temperatur von 37°C einem Auflösungstest unterzogen. Dabei wurde in einem Prüfvolumen von 900 ml zunächst durch Zugabe von 0,08 N Salzsäure ein pH-Wert von 1,2 eingestellt. Nach einem Zeitraum von 2 h wurde das Prüfmedium mit Hilfe der Pufferadditionsmethode auf einen pH-Wert von 6,8 umgestellt. Zur Messung der Konzentration des freigesetzten Wirkstoffes wurde von Anfang an, also auch schon bei pH 1,2, stündlich ein Probenvolumen von 10 ml entnommen und die optische Extinktion bei einer Wellenlänge von 270 nm mit Hilfe eines Spektralphotometers gemessen. Die Ergebnisse des Auflösungstests sind in Tab. 1 aufgeführt.

### Beispiel 2

Beschichtung wie bei Beispiel 1, jedoch Beschichtungszeit 56 min.

### Beispiel 3

Aus Pellets des Wirkstoffes Theophyllin wurde durch Siebung eine Fraktion mit einem Pelletdurchmesser zwischen 1,0 und 1,4 mm ausgewählt. Die Wirkstoffträger wurden mit Ethylcellulose, gelöst in einem Aceton/Isopropanol-Gemisch, in einer Monolage auf eine PET-Folie aufgeklebt.

Die Folie mit den Wirkstoffträgern wurde in eine zylinderförmige Vakuumanlage eingebracht, welche mit einem zweistufigen Pumpsystem (Turbomolekularpumpe und Drehschiebervorpumpe) auf einen Druck von 10⁻⁶ mbar evakuiert wurde. Danach wurde über eine ringförmige Gasdusche mit einem Innendurchmesser von 150 mm ein Gasgemisch aus Hexamethyldisiloxan mit einem Partialdruck von 2 x 10⁻² mbar und Argon mit einem Partialdruck von 1 x 10⁻² mbar eingelassen. Bei konstanter Saugleistung des Pumpsystems betrug dabei der Fluß von Hexamethyldisiloxan 35,8 sccm.

Eine zentrisch gegenüber der Trägerfolie in einem Abstand von 56 mm angebrachte runde Stahlkathode (Durchmesser 150 mm) wurde mit Hilfe eines RF-Generators mit einer Wechselspannung der Frequenz 13,56 MHz beaufschlagt. Die Leistungsabgabe des RF-Generators wurde auf 100 W geregelt, wobei sich auf der Stahlelektrode ein Potential von -480 V gegenüber einem geerdeten Bezugspunkt einstellte. Nach einer Beschichtungszeit von 6 min 12 s wurde der RF-Generator abgeschaltet und damit die Beschichtung beendet.

Die solcherart beschichteten Pellets wurden im aufgeklebten Zustand einem Auflösungstest wie in Beispiel 1 unterzogen.

Die Ergebnisse des Auflösungstests sind in Tab. 1 aufgeführt.

### Beispiel 4

Wie Beispiel 3, jedoch Beschichtungszeit 12 min 24 s.

### Beispiel 5

Wie Beispiel 3, jedoch Beschichtungszeit 49 min 38 s.

**Tabelle 1**

| Auflösungszeit [h] | freigesetzte Wirkstoffmenge [%] | | | | | |
|---|---|---|---|---|---|---|
| | Vergleichsprobe (unbeschichtet) | Beispiele | | | | |
| | | 1 | 2 | 3 | 4 | 5 |
| 0 | 0 | 0 | 0 | 0 | 0 | 0 |
| 1 | 98,4 | 67,5 | 17,3 | 8,4 | 3,5 | 0,4 |
| 2 | 99,5 | 84,5 | 28,3 | 19,6 | 8,8 | 2,4 |
| 3 | 100 | 90,0 | 36,8 | 30,6 | 13,4 | 3,0 |
| 4 | 100 | 93,5 | 43,9 | 42,1 | 19,6 | 4,0 |
| 5 | 100 | 95,2 | 50,2 | 50,7 | 25,1 | 5,5 |
| 6 | 100 | 96,7 | 55,6 | 59,9 | 29,5 | 6,2 |
| 7 | 100 | 98,2 | 60,3 | 70,8 | 35,1 | 7,6 |
| 8 | 100 | 98,3 | 63,8 | 77,2 | 40,5 | 9,0 |

## Patentansprüche

1. Retardform für wasserlösliche, feste pharmazeutische Wirkstoffe, bestehend aus einem den pharmazeutischen Wirkstoff enthaltenden oder daraus bestehenden Kern und mindestens einer diffusionshemmenden, haftfesten Deckschicht, dadurch gekennzeichnet, daß die Deckschicht aus einem chemischen Netzwerk mit einer Dicke im Bereich von 0,01 bis 10 µm besteht, das in einem plasmaunterstützten chemischen Abscheideverfahren auf den Träger aufgebracht wurde.

2. Retardform nach Anspruch 1, dadurch gekennzeichnet, daß die Deckschicht mindestens die Elemente Silizium und Kohlenstoff enthält, wobei der Si-Gehalt der Deckschicht im Bereich von 1 bis 40 Atom-% und der C-Gehalt im Bereich von 60 bis 99 Atom-% (bezogen jeweils auf den Gesamtgehalt aller Elemente außer Wasserstoff) liegt.

3. Retardform nach Anspruch 1 oder 2, dadurch gekennzeichnet, daß der Kern aus einem Granulat, Agglomerat oder einem Kristall des Wirkstoffes besteht.

4. Retardform nach Anspruch 1 oder 2, dadurch gekennzeichnet, daß der Kern aus einem Pellet besteht, welches den Wirkstoff sowie pharmazeutische Hilfsstoffe enthält.

5. Retardform nach Anspruch 1 oder 2, dadurch gekennzeichnet, daß der Kern aus einer Tablette besteht, welche den Wirkstoff sowie pharmazeutische Hilfsstoffe enthält.

6. Verfahren zur Herstellung einer Retardform nach einem der Ansprüche 1 bis 5, dadurch gekennzeichnet, daß die diffusionshemmende Deckschicht durch plasmaunterstützte Zersetzung eines Gasgemisches, welches mindestens eine organische Verbindung enthält, in einem abgeschlossenen Volumen unter Abscheidung und gleichzeitiger Netzwerkbildung auf den in Bewegung gehaltenen Wirkstoffträgern erzeugt wird.

7. Herstellungsverfahren nach Anspruch 6, dadurch gekennzeichnet, daß das Gasgemisch mindestens eine Si-organische Verbindung enthält.

8. Herstellungsverfahren nach Anspruch 7, dadurch gekennzeichnet, daß als Si-organische Verbindung Hexamethyldisiloxan verwendet wird.

9. Herstellungsverfahren nach einem der Ansprüche 6 bis 8, dadurch gekennzeichnet, daß der Fluß im Bereich von 1 bis 10.000 sccm bei einem Druck im Bereich von 1 x 10⁻³ bis 100 mbar eingestellt wird.

## Claims

1. A delayed-release form for water-soluble, solid pharmaceutical active compounds, consisting of a core containing the pharmaceutical active compound or consisting thereof and at least one diffusion-inhibiting, adhesive coating layer, wherein the coating layer consists of a chemical network having a thickness in the range from 0.01 to 10 µm, which has been applied to the support in a plasma-assisted chemical deposition process.

2. A delayed-release form as claimed in claim 1, wherein the coating layer contains at least the elements silicon and carbon, the Si content of the coating layer being in the range from 1 to 40 atom % and the C content in the range from 60 to 99 atom % (in each case based on the total content of all elements apart from hydrogen).

3. A delayed-release form as claimed in claim 1 or 2, wherein the core consists of granules, an agglomerate or a crystal of the active compound.

4. A delayed-release form as claimed in claim 1 or 2, wherein the core consists of a pellet which contains the active compound and pharmaceutical auxiliaries.

5. A delayed-release form as claimed in claim 1 or 2, wherein the core consists of a tablet which contains the active compound and pharmaceutical auxiliaries.

6. A process for preparing a delayed-release form as claimed in one of claims 1 to 5, which comprises producing the diffusion-inhibiting coating layer by plasma-assisted decomposition of a gas mixture which contains at least one organic compound in a closed volume with deposition and simultaneous network formation on the active compound supports, which are kept in motion.

7. A preparation process as claimed in claim 6, wherein the gas mixture contains at least one organosilicon compound.

8. A preparation process as claimed in claim 7, wherein the organosilicon compound used is hexamethyldisiloxane.

9. A preparation process as claimed in one of claims 6 to 8, wherein the flow rate is set in the range from 1 to 10,000 sccm at a pressure in the range from 1 × 10⁻³ to 100 mbar.

## Revendications

1. Forme-retard pour principes actifs pharmaceutiques solides solubles dans l'eau, constituée d'un noyau, contenant le principe actif pharmaceutique ou bien étant constituée de celui-ci, et d'au moins une couche de couverture, fixée par adhésion, empêchant la diffusion, caractérisée par le fait que la couche de couverture est constituée d'un réseau chimique ayant une épaisseur dans la plage de 0,01 à 10 µm, qui a été appliquée sur le support selon un procédé de déposition chimique assisté par plasma.

2. Forme-retard selon la revendication 1, caractérisée par le fait que la couche de couverture contient au moins les éléments que sont le silicium et le carbone, la teneur en Si de la couche de couverture étant située dans la plage allant de 1 à 40 % en atomes et la teneur en C dans la plage de 60 à 99 % en atomes (en se référant chaque fois à la teneur totale de tous les éléments hors l'hydrogène).

3. Forme-retard selon la revendication 1 ou 2, caractérisée par le fait que le noyau est constitué d'un granulat, d'un agglomérat ou bien d'un cristal du principe actif.

4. Forme-retard selon la revendication 1 ou 2, caractérisée par le fait que le noyau est constitué d'une boulette qui contient le principe actif ainsi que des substances auxiliaires pharmaceutiques.

5. Forme-retard selon la revendication 1 ou 2, caractérisée par le fait que le noyau est constitué d'un comprimé qui contient le principe actif ainsi que des substances auxiliaires pharmaceutiques.

6. Procédé de fabrication d'une forme-retard selon les revendications 1 à 5, caractérisé par le fait que la couche de couverture empêchant la diffusion est produite par décomposition, assistée par plasma, d'un mélange de gaz qui contient au moins une combinaison organique, dans un volume fermé avec précipitation et simultanément formation d'un réseau sur le support de principe actif maintenu en déplacement.

7. Procédé de fabrication selon la revendication 6, caractérisé par le fait que le mélange de gaz contient au moins une combinaison organique de Si.

8. Procédé de fabrication selon la revendication 7, caractérisé par le fait qu'on utilise, comme combinaison organique de Si, de l'hexaméthyldisiloxane.

9. Procédé de fabrication selon une des revendications 6 à 8, caractérisé par le fait que le flux est réglé dans la plage allant de 1 à 10000 Ncm³/mn pour une pression comprise dans la plage de 1 x 10-3 à 100 mbar.
